Europäisches Patentamt

European Patent Office

Office européen des brevets

(19)

(11) Publication number: **0 265 864 B1**

# EUROPEAN PATENT SPECIFICATION

(12)

(45) Date of publication of patent specification: **02.01.92**  (51) Int. Cl.⁵: **A61M  25/00**

(21) Application number: **87115555.2**

(22) Date of filing: **23.10.87**

(54) Hemostasis sheath.

(30) Priority: **30.10.86 US 925010**
    **22.05.87 US 53178**
    **22.05.87 US 53183**

(43) Date of publication of application:
    **04.05.88 Bulletin  88/18**

(45) Publication of the grant of the patent:
    **02.01.92 Bulletin  92/01**

(84) Designated Contracting States:
    **BE CH DE ES FR GB IT LI NL SE**

(56) References cited:
    **US-A- 3 421 509**
    **US-A- 4 327 709**
    **US-A- 4 422 447**
    **US-A- 4 473 067**

(73) Proprietor: **KONTRON INSTRUMENTS HOLD-
ING N.V.
Pietermaai 6 A P.O. Box 504
Curacao(AN)**

(72) Inventor: **Cho, George Eng Sao
19 Elsbeth Road
Sudbury Massachusetts 01776(US)**
Inventor: **Cicciu, Gerald Joseph
182 Wayside Inn Road
Sudbury Massachusetts 01776(US)**
Inventor: **Lombardi, Edward James
48 Lawrence Road
Derry New Hampshire 03038(US)**

(74) Representative: **Riccardi, Sergio
Riccardi & Co. Via Macedonio Melloni, 32
I-20129 Milano(IT)**

EP 0 265 864 B1

## Description

The present invention relates to an intra aortic balloon apparatus including a balloon catheter having a proximal and a distal end and an inflatable and deflatable balloon bladder sealidly attached at the distal end of the catheter and a method of inserting same into the body. In particular, the invention relates to a hemostasis sheath used as a component of an IAB device. When used in combination with a tear away insertion sheath, the hemostasis sheath facilitates insertion of the IAB device into the body by lowering the degree of obstruction in the femoral artery while controlling bleeding back through the insertion site after the tear away insertion sheath is removed. The IAB device of this invention may also be advantageously used in a sheathless insertion technique described herein. When used in combination with the sheathless insertion technique, the hemostasis sheath facilitates use of the IAB device by lowering the degree of obstruction in the femoral artery while controlling bleeding back through the insertion site after the IAB has been inserted.

IAB devices are introduced into the body and are used to assist the pumping action of the heart. See, for example, U.S. Patent No. 4,362,150. In some instances, they may remain in the body for extended periods of time, such as several days or more.

One method of installing an IAB device in the body is via a non-surgical insertion into the femoral common artery through the skin using the percutaneous insertion (Seldinger) technique.

In the prior art percutaneous insertion technique, the skin is punctured to form a hole through the skin and into the femoral artery. A short guide wire is inserted into the femoral artery and the hole is then expanded by an inserter dilator (for example, an 8-French dilator) which slides over the guide wire through the skin and into the artery. The inserter dilator is removed and a series of progressively larger dilators are inserted into the hole over the guide wire to increase the size of the hole. Next an insertion sheath is passed through the hole and into the femoral artery. This sheath has an inside diameter generally corresponding to the outside diameter of the IAB to be inserted. The short guide wire is removed and is replaced by a long guide wire which is fed up through the artery to the vicinity of the aorta. The IAB is passed over the guide wire and slides up through the sheath and along the artery all the way up to the aorta.

Although the foregoing procedure generally is a safe, rapid and efficacious way of intra-aortic balloon insertion, the prerequisite insertion and use of the sheath is a step which requires time and equipment to perform, often under circumstances such that time is a critical factor to patient survival, as during cardiogenic shock.

During the foregoing described procedure, arterial bleeding through the sheath must be carefully controlled during the time interval between the removal of the short guide wire from the sheath and the insertion of the wrapped balloon over the long guide wire. Often, especially in a hypovolemic patient, this loss of blood may be critical. Also when the balloon bladder is wrapped, spiral interstices are produced along its length. The interstices of the wrapped balloon membrane do not provide for the complete occlusion of the sheath between its inner wall and the wrapped balloon. Therefore, a certain amount of arterial bleeding takes place during the time that is required to fully insert the wrapped balloon membrane portion of the balloon catheter into the blood vessel.

In some cases, the sheath may have to be withdrawn partly from the percutaneous wound to permit complete introduction of the balloon membrane into the sheath, especially in those cases of extreme vascular tortuosity. This creates an additional loss of critical time and of critical blood.

Another problem experienced with some patients, is that after IAB insertion is complete, blood flow to the lower extremities is diminished substantially. The decrease in blood flow is generally attributable to the obstruction of the femoral artery caused by the relatively large diameter of the insertion sheath extending into the artery. By removing the sheath, the obstruction in the femoral artery can be decreased substantially. Certain prior art techniques attempt to solve this concern by utilizing splittable ("tear away") insertion sheaths. Various types of tear away, removable insertion sheaths which would be suitable for this purpose are disclosed in the prior art. See for example, U.S. Patent Nos. 4,166,469, 4,581,019 and 4,581,025. Once the insertion sheath is removed, there would only remain the IAB catheter which connects an inflatable and deflatable bladder member of the IAB with the external pumping/monitoring equipment.

U.S. Patent No. 4,540,404 attempts to address these concerns by using an IAB with a tapered tip and a sheath which slides over the balloon bladder to form an assembly. After insertion, the sheath can be withdrawn to expose the balloon.

However, after removal of the insertion sheath the arterial wall must constrict to seal around the balloon catheter, which has a smaller outside diameter than the insertion sheath. Therefore, in nonelastic or diseased vessels, the required vessel constriction may not always occur resulting in profuse bleeding from the insertion site between the IAB catheter and arterial puncture.

One way to stop this bleeding is to exert pressure on the artery above the insertion site. However, this has the disadvantage that it may damage the balloon catheter and requires time and an additional step in the IAB insertion process. If bleeding cannot be stopped, the IAB must be removed.

US-A-4 422 447 discloses an intra aortic balloon apparatus including a balloon catheter having a proximal and a distal end and an inflatable and deflatable balloon bladder sealingly attached at the distal end of the catheter, comprising: a hemostasis sheath slidably coupled with the balloon catheter, between the balloon bladder and the proximal end of balloon catheter, said hemostasis sheath having a distal end adjacent to the balloon bladder, and sealing means releasably coupled to said hemostasis sheath for preventing bleeding between said hemostasis sheath and said balloon catheter after insertion of the balloon bladder in the patient. However, the sealing means disclosed in this prior art patent is constituted by a flange at the proximal end of the hemostasis sheath, and this flange resulted to be insufficient to prevent said bleeding.

Starting from an apparatus of the kind disclosed in US-A-A 422 447, these problems are solved by an intra aortic balloon apparatus having the features recited in the characterising part of Claim 1.

The IAB apparatus according to the invention can be inserted into the patient using either a tear away insertion sheath or using a sheathless insertion technique as described herein. The balloon catheter has a hemostasis sheath slidably coupled thereon above the balloon bladder and sealed by sealing means at its proximal end. The hemostasis sheath has a smaller diameter at its distal end and a larger diameter at its proximal end so that it can be slid into the insertion site after IAB insertion until the point is reached where its increased diameter fills the opening so as to stop bleeding.

A preferred embodiment of the invention will now be described with reference to the following drawings, which are merely exemplary and are not meant to limit the scope of the invention in any respects.

Figs. 1a-d show in succession (a) the puncture of the skin and artery, (b) insertion of the guide wire, (c) dilation of the insertion site and (d) insertion of an insertion sheath employing a prior art (Seldinger) technique.

Fig. 2a is a side elevation view of an IAB device showing the IAB bladder being directly inserted into the femoral common artery without an insertion sheath according to the inventive method:

Fig. 2b is a side elevation view partly in cross-section showing the IAB device of Fig. 1 following insertion of the IAB bladder into the femoral common artery;

Fig. 3 is a side elevation view of the IAB device of Figs. 1 and 2 showing the hemostasis sheath of the present invention positioned within the insertion puncture;

Fig. 4 is a side elevation view of an embodiment of an IAB device according to the present invention showing the IAB bladder being inserted into the femoral common artery;

Fig. 5 is a side elevation view partly in cross-section showing the IAB device of Fig. 4 following insertion of the IAB bladder into the femoral common artery;

Fig. 6 is a side elevation view of the IAB device of Figs. 4 and 5 showing the hemostasis sheath of the present invention positioned within the insertion puncture;

Fig. 7 is a cross-section of the hemostasis sheath according to the invention showing the hemostasis sheath installed on a balloon catheter;

Fig. 8 is a cross-section of another embodiment of the hemostasis sheath according to the invention; and

Fig. 9 is a cross-section of yet another embodiment of the hemostasis sheath according to the invention showing an inside diameter which gradually increases from the distal end to the proximal end.

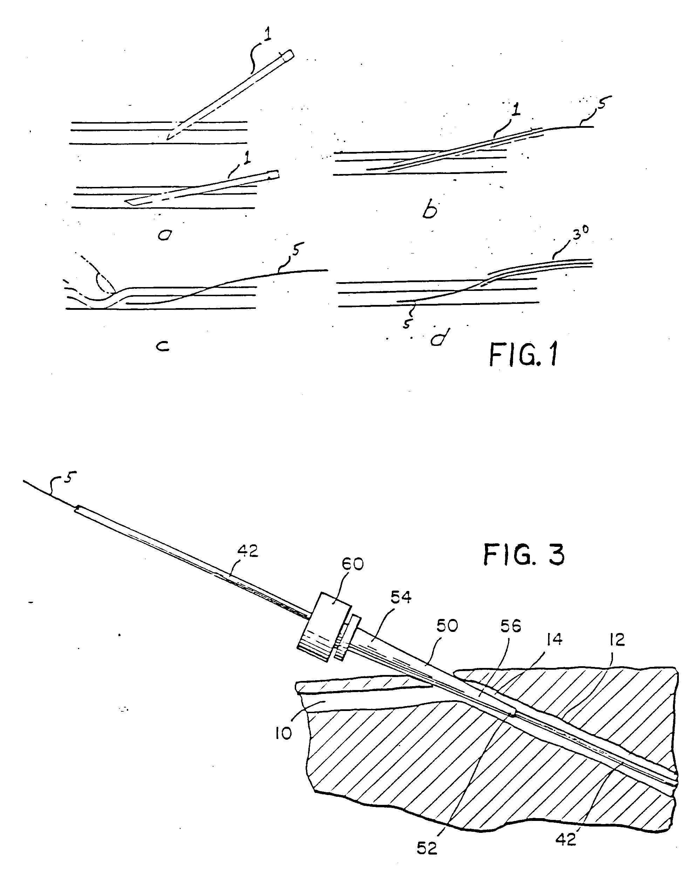

Figs. 1a-1d show various steps employed in the prior art (Seldinger) technique for inserting an IAB device percutaneously. Fig. 1a shows puncture of the skin and the femoral artery using a hypodermic needle (e.g. Potts cone head). Fig. 1b shows placement of a guide wire 5 into the artery through the hollow bore of the needle. Fig. 1c shows removal of the hypodermic needle from the artery leaving the guide wire 5 in place. Finally, Fig. 1d shows placement of an insertion sheath into the artery over the guide wire following dilation of the insertion site.

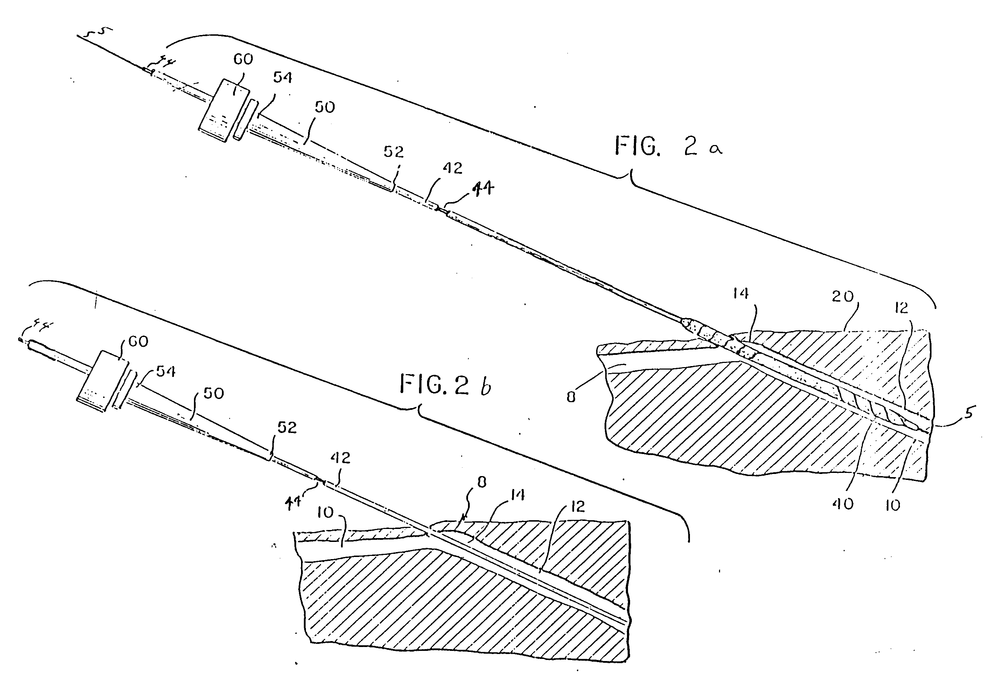

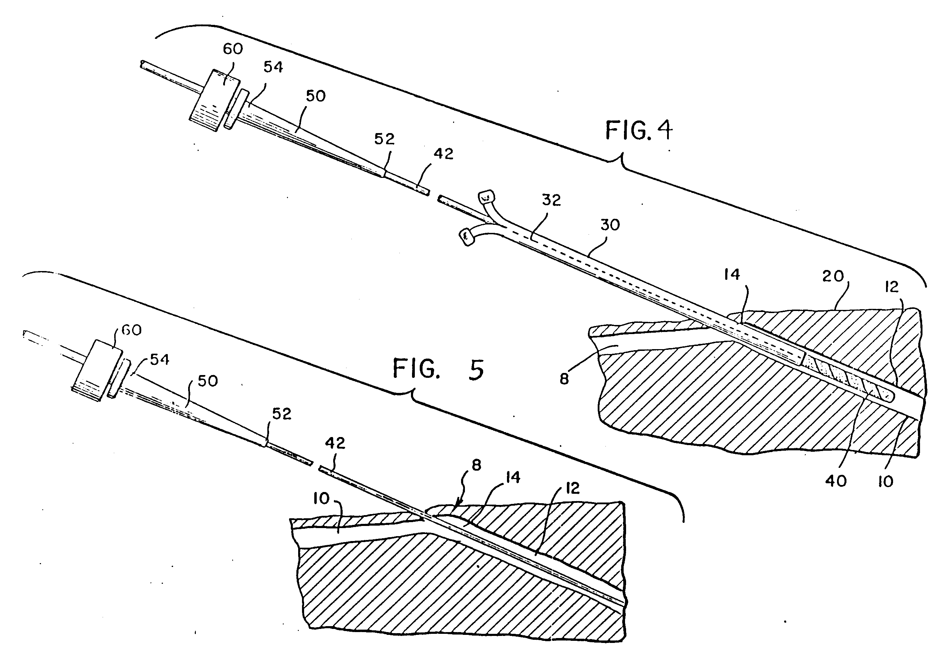

With reference to Figs. 2a, 2b, and 3 the insertion of an IAB device into the body via a non-surgical insertion into the femoral common artery through the skin using a new percutaneous insertion technique according to the invention will be described. A physician (not shown) would be positioned in the left-hand margin in relation to the various elements being described. The terms "proximal" and "distal" as used herein shall refer to position relative to that of the physician.

Referring to Figs. 2a and 2b, the IAB device generally comprises IAB bladder 40 which is attached to balloon catheter 42. The IAB is a double lumen device with a central hollow stylette 44 and preferably of the type described in US Patent No. 4,362,150, which patent is incorporated herein by reference. The IAB can have a wrap handle for rotation as described in the above patent or can have a fixed type configuration. The hollow stylette preferably is a hypodermic tubing with a flexible segment within the balloon.

Prior to insertion, the bladder 40 is wrapped about itself to reduce its diameter either by the manufacturer or by the physician. The balloon catheter 42, for example, as is known in the art may be attached at its proximal end to a rotating or fixed handle (not shown) and may also be connected in known manner to an intra aortic balloon pumping/monitoring system (also not shown).

An insertion technique for the apparatus according to the invention will now be described.

With reference to Fig. 2a, a small hypodermic needle (not shown) is inserted through the skin 20 of a patient to perforate or puncture the femoral artery 10. When blood spurts from the open external end of the needle, placement of the hypodermic needle within the artery 10 is confirmed. A long guide wire 5 (e.g. up to about 150 - 190 cm or longer) sufficient in length to reach the central aorta is fed into the artery 10 by passing the guide wire through the center of the hollow hypodermic needle.

Next, the hypodermic needle is removed leaving the guide wire 5 in place. One or more progressively larger dilators (preferrably a single expanding, e.g. Grunzig type dilator) is then placed over the guide wire and advanced through the perforated skin 20 and into the artery 10 in order to expand the hole in order to achieve an opening large enough to permit the passage of the wrapped IAB bladder 40. For example, when using a 10.5 French IAB the hole should be dilated to approximately 10 French in diameter. Once the skin 20 and artery 10 have been fully dilated, the dilator is removed and the IAB device is inserted directly into the patient without passing it through an insertion sheath.

Still referring to Fig. 2a, the IAB bladder 40 even in its wrapped condition has a larger outside diameter than the IAB catheter 42. As a result the IAB bladder 40 will dilate the insertion site to a larger diameter than that of the catheter 42.

Reference is now made to Fig. 2b which shows, from left to right, the hemostasis sheath 50 and IAB catheter 42 of Figure 2a with the IAB bladder 40 now inserted into the aorta (not shown).

As can be seen in Fig. 2b, the insertion site 8 after passage of the IAB, may have an opening which due to some inelasticity in the skin has not completely closed around the catheter 42. This condition may result in uncontrollable bleeding from the insertion site 8.

As a means to diminish this bleeding when it occurs, the present invention utilizes hemostasis sheath 50 which is slidably coupled to the catheter 42.

As will be discussed in more detail below, the hemostasis sheath 50 preferably has a conical configuration and has a distal end 52 which in the preferred embodiment is only slightly larger than the outside diameter of the catheter 42. Preferably, the inside of the distal end 52 is sized for a close fit over the outside of catheter 42. The hemostasis sheath 50 also has a proximal end 54 opposite from the distal end 52. The proximal end 54 has a larger outside diameter than the distal end 52. Preferably, the proximal end 54 has an outside diameter which is about at least as large or slightly larger than the outside diameter of the IAB bladder 40 in its wrapped condition.

Fig. 3 shows from left to right the IAB catheter 42 and hemostasis sheath 50 of Figures 2a and 2b with the hemostasis sheath 50 now positioned in the insertion site 8.

With reference to Fig. 3, the hemostasis sheath 50 has now been inserted partially into the opening 14 in the wall of the artery 10 with its distal end 52 extending inside the artery 10. The hemostasis sheath 50 is inserted into the artery 10 until the point is reached where its increased diameter at point 56, between the distal and 52 and proximal end 54, fills the opening 14. As shown in Fig. 3, the hemostasis sheath 50 is thereby able to stop the bleeding which might have resulted after insertion of the IAB device. Additionally, the hemostasis sheath 50 is configured and dimensioned to pass through the skin 20 and into the artery 10, and is able to control bleeding without restricting good blood flow through the artery 10 to any great degree.

In accordance with this insertion technique, the hemostasis sheath 50 is advanced along the balloon catheter 42 through the skin and into the artery by a sufficient distance to control bleeding from the insertion site 8. In particular, the hemostasis sheath 50 is advanced to a point where its outside diameter sufficiently fills the opening made by the passage of the IAB bladder through the skin and artery to provide an elastic contact between the skin opening and the outside diameter of the hemostasis sheath 50.

An alternative IAB insertion technique for the apparatus according to the invention using a tearaway insertion sheath will now be described with reference to Figs. 4-6.

Fig. 4 shows, from left to right, a hemostasis sheath 50, an IAB catheter 42, a tear away insertion sheath 30, an IAB bladder 40 and femoral common artery 10.

With reference to Fig. 4, an IAB device is shown being installed in the body via a non-surgical insertion into the femoral common artery 10 through skin 20 using the percutaneous insertion (Seldinger) technique. The IAB device generally comprises IAB bladder 40 which is attached to balloon catheter 42. Prior to insertion, the bladder 40 is wrapped about itself to reduce its diameter. The balloon catheter 42 may be attached at its proximal end to a wrap handle (not shown) and may also be connected to an intra aortic balloon pumping/monitoring system (also not shown).

As shown in Fig. 4, tear away insertion sheath 30 is installed in the artery 10 and extends out through an opening 14. Intra aortic balloon 40 is being passed through the tear away insertion sheath 30 and into the artery 10. The balloon catheter 42 can be inserted into the artery 10 using the following percutaneous insertion (Seldinger) technique:

A small hypodermic needle (not shown) is inserted through the skin 20 of a patient to perforate or puncture the artery 10. When blood spurts from the open external end of the needle, placement of the hypodermic needle within the artery 10 is confirmed. A guide wire (also not shown) is fed into the artery 10 by passing the guide wire through the center of the hollow hypodermic needle. The hypodermic needle is then removed leaving the wire in place. A dilator (also not shown) is then placed over the guide wire and advanced through the perforated skin 20 and into the artery 10 in order to dilate (that is enlarge) the artery 10 and create opening 14. The dilator is then removed and a series of larger dilators (not shown) are then fed over the guide wire and into the artery 10 to continue the dilation procedure. Once the artery 10 has been fully dilated, the insertion sheath 30 is inserted through the opening 14 and into the artery and the last dilator is removed leaving the insertion sheath 30 extending through the opening 14 and available for insertion of the IAB device over the guide wire and into the patient without requiring surgery.

The tear away insertion sheath 30 may have a tear line 32, which permits the insertion sheath 30 to be torn therealong and removed from insertion site 8 once the balloon 40 has been inserted into the aorta. Alternatively, the tear away insertion sheath 30 may be formed by linear extrusion (as is known in the art) to facilitate tearing thereof for removal purposes.

The tear away insertion sheath 30 then is removed. However, removal of the insertion sheath 30 may result in uncontrolled bleeding from the insertion site 8.

As a means to diminish this bleeding when it occurs, the present invention as described above provides hemostasis sheath 50 which is slidably coupled to the catheter 42.

Fig. 5 shows, from left to right, the hemostasis sheath 50 and IAB catheter 42 of Figures 2a, 2b, 3 and 4 with the IAB bladder 40 now inserted into the aorta (not shown). The tear away insertion sheath 30 (also not shown) has been removed at this point.

Additionally, as shown in Fig. 5, the wall of the femoral artery 12 has not completely constricted around the balloon catheter 42. As a result, uncontrolled bleeding from the insertion site 8 might be occurring at this point. In order to control the bleeding, the hemostasis sheath 50 is slid down the balloon catheter 42 in the direction of the arrow towards the insertion site 8 and partially into the opening 14.

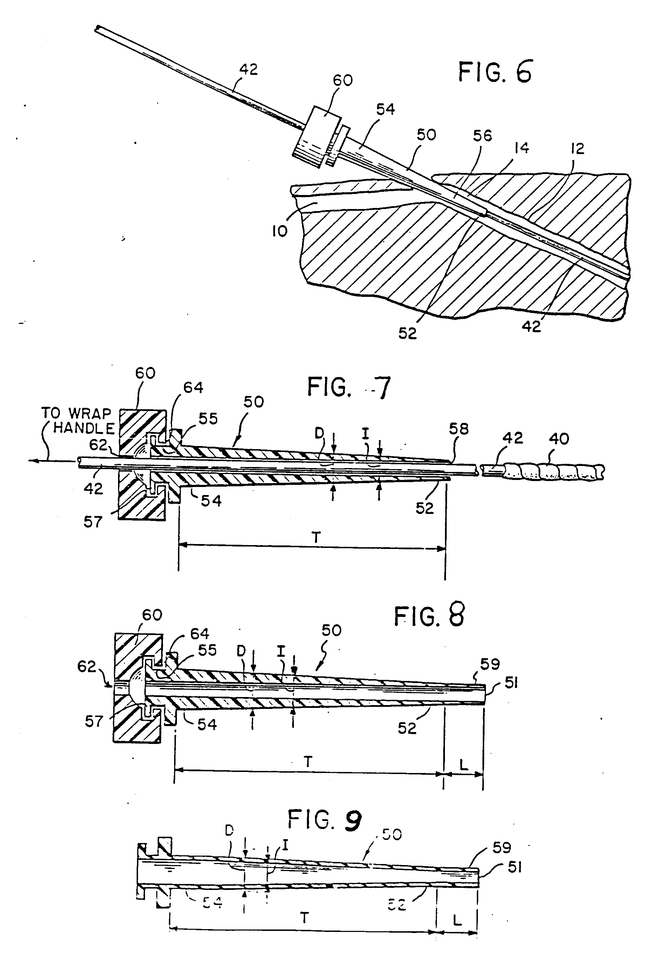

Fig. 6 shows from left to right the IAB catheter 42 and hemostasis sheath 50 of Figures 2a, 2b, 3, 4 and 5 with the hemostasis sheath 50 now positioned in the insertion site 8.

With reference to Fig. 6, the hemostasis sheath 50 has now been inserted partially into the opening 14 in the wall of the artery 10 with its distal end 52 extending inside the artery 10. The hemostasis sheath 50 is inserted into the artery 10 until the point is reached where its increased diameter at point 56, between the distal end 52 and proximal end 54, fills the opening 14. As shown in Fig. 6, the hemostasis sheath 50 is thereby able to stop the bleeding which might have resulted when the insertion sheath 30 was removed. Additionally, the hemostasis sheath 50 is configured and dimensioned to be less obstructive than the insertion sheath 30 within the artery 10, and is therefore able to control bleeding without restricting good blood flow through the artery 10 to any great degree in comparison with the insertion sheath 30.

The hemostasis sheath 50 is shown in greater detail in Figs. 7, 8 and 9. Unless otherwise indicated, Figs. 7, 8 and 9 show, from left to right the IAB catheter 42 (Fig. 4 only) a cuff 60 and hemostasis sheath 50. Additionally, from left to right, the hemostasis sheath comprises a flange 57, a neck 55, proximal end 54, distal end 52 and an extended portion 59 including a tip 51 (Fig. 8 only).

The outside diameter (D) of the hemostasis sheath 50 gradually increases from its distal end 52 towards its proximal end 54. The inside diameter (I) of the hemostasis sheath 50 may be about the same throughout its entire length (as shown in Figs. 7 and 8) or may gradually increase from the distal end 52 towards the proximal end 54 (as shown in Fig. 6, for example). Preferably, the inside diameter (I) is sized at the distal end 52 to provide a close clearance 58 between the inside of the hemostasis sheath 50 and the outside of the balloon catheter 42. Preferably the inside diameter (I) of the hemostasis sheath 50 at its distal end 52 is between about 50 to 76 $\mu$m (2 to 3 thousandths of an inch) larger than the outside diameter of the balloon catheter 42 to allow for manufacturing tolerance. More preferably, in view of the resilience of the materials utilized, one can maintain an interference fit such that at the distal end 52 the inside diameter (I) of the hemostasis sheath 50 is the same as the outside diameter of the balloon catheter 42. This close fit clearance 58 permits the outside diameter of the hemostasis sheath 50 to be as small as possible at the distal end 52 with the balloon catheter 42 providing structural support for the hemostasis sheath 50 during insertion to prevent an accordion effect from occurring at the distal end 52. This close fit clearance also reduces the risk of bleedblock between the bladder catheter 42 and the hemostasis sheath 50.

For example, in a preferred embodiment wherein the outside diameter of the balloon catheter is about 10.5 french (i.e., about 3.50 mm or 0.138 inches), the outside diameter (D) of the hemostasis sheath 50 at its distal end 52 is about 3.70 mm (0.146 inches) the outside diameter (D) at its proximal end 54 is about 4.70 mm (0.185 inches) and the inside diameter (I) of the hemostasis sheath 50 at the distal end 52 is about 3.55 mm or 0.140 inches to provide a clearance 58 of about 50 $\mu$m (0.002 inches) at the distal end 52.

Preferably, the outside diameter (D) of the hemostasis sheath 50 tapers gradually from its distal end 52 to its proximal end 54 and the distance (T) between the distal end 52 and proximal end 54 is preferably about 5 to 6.35 cm (2.0 to 2.5 inches). Of course, the outside diameter may also increase in a step-wise manner (not shown). By "gradual" is meant any shape which increases in outside diameter in a continuous, as opposed to step-wise, manner. Such gradual shapes include cones having straight sides along their length or curved sides.

As shown in Figs. 8 and 9, in another preferred embodiment, the distal end 52 of the hemostasis sheath 50 may further include constant diameter portion 59.

The constant diameter portion 59 would preferably have a length of at least about 6.35 mm (1/4 inch) and an inside diameter which is about 50 to 76 $\mu$m (2 to 3 thousandths) of an inch) larger than the outside diameter of the balloon catheter 42.

The constant diameter portion 59 is configured to generate less initial resistance during insertion through the skin so that the tip 51 of the hemostasis sheath 50 can be more easily inserted. Once the constant diameter portion 59 has been inserted through the skin, the overall resistance to further insertion will increase as the outside diameter of the hemostasis sheath 50 increases. However, with the tip 51 safely passed through the skin, the danger of collapsing or buckling as an accordian at the tip 51 can be avoided.

The hemostasis sheath 50 is provided at its proximal end with a neck 55 and flange 57. The neck 55 and flange 57 are held within the cuff 60. As shown in Figs. 7 and 8, the cuff 60 is sized to provide a close clearance 64 between the cuff 60 and neck 55 of the hemostasis sheath 50. Additionally, the cuff 60 is sized to provide a close clearance 62 between the cuff 60 and the balloon catheter 42. In this manner, the cuff 60 is able to seal the proximal end of the hemostasis sheath 50 against bleeding when the distal end 52 is positioned within the femoral artery 10. Close clearance 62 also precludes slippage of cuff 60 and, in turn, hemostasis sheath 50 along catheter 42 due to arterial pressure and the like.

In a preferred embodiment, the hemostasis sheath 50 is manufactured from resilient, for example a plastic, material, which is preferably polytetrafluoroethylene (Teflon®) or polyethylene. Also in the preferred embodiment, the cuff 60 is manufactured from an elastomeric material, for example silicone. However, no particular elastomeric material is preferred.

The various features and advantages of the invention are thought to be clear from the foregoing description. Various other features and advantages not specifically enumerated will undoubtedly occur to those versed in the art as likewise will many variations and modifications of the preferred embodiment illustrated, all of which may be achieved without departing from the spirit and scope of the invention as defined by the following claims.

## Claims

1. Intra aortic balloon apparatus including a balloon catheter (42) having a proximal and a distal end and an inflatable and deflatable balloon bladder (40) sealingly attached at the distal end of the catheter, comprising: a hemostasis sheath (50) slidably coupled with the balloon catheter, between the balloon bladder (40) and the proximal end of balloon catheter (42), said hemostasis sheath (50) having a distal end (52) adjacent to the balloon bladder (40), and sealing means (60) releasably coupled to said hemostasis sheath (50) for preventing bleeding between said hemostasis sheath (50) and said balloon catheter (42) after insertion of the balloon bladder (40) in the patient, characterised in that the hemostasis sheath (50) has an outside diameter which is gradually increased from the distal end (52) to the proximal end (54) of said hemostasis sheath (50).

2. Apparatus of claim 1, characterized in that the distal end of said hemostasis sheath (50) includes a portion (59) of constant diameter extending towards said balloon bladder (40).

3. Apparatus of claim 2, characterized in that the constant diameter portion (59) has a length of at least about 6.35 mm (one-quarter of an inch).

4. Apparatus of claim 3, characterized in that said hemostasis sheath (50) has a conical configuration.

5. Apparatus of claim 1, characterized in that the hemostasis sheath (50) has an outside diameter which increases from the distal end (52) to the proximal end (54) of said hemostasis sheath (50).

6. Apparatus of claim 5, characterized in that said hemostasis sheath (50) has an inside diameter which is slightly larger than the outside diameter of the balloon catheter (42) with a clearance (58) therebetween of no more that about 50 to 76 $\mu$m (2 to 3 thousandths of an inch) at least at the distal end (52) of said hemostasis sheath (50).

**Revendications**

1. Appareil à ballonnet intra-aortique incluant un cathéter à ballonnet (42) ayant une extrémité proximale et une extrémité distale et une vessie de ballonnet gonflable et dégonflable (40) fixée de manière étanche à l'extrémité distale du cathéter, comprenant: une gaine d'hémostase (50) couplée de manière à pouvoir glisser avec le cathéter à ballonnet, entre la vessie du ballonnet (40) et l'extrémité proximale du cathéter à ballonnet (42), ladite gaine d'hémostase (50) ayant une extrémité distale (52) adjacente à la vessie du ballonnet (40), et un joint d'étanchéité (60) couplé de manière libérable à ladite gaine d'hémostase (50) pour empêcher tout saignement entre ladite gaine d'hémostase (50) et ledit cathéter à ballonnet (42) après insertion de la vessie à ballonnet (40) chez le malade, caractérisé en ce que la gaine d'hémostase (50) a un diamètre externe qui s'accroît graduellement de l'extrémité distale (52) à l'extrémité proximale (54) de ladite gaine d'hémostase (50).

2. Appareil de la revendication 1, caractérisé en ce que l'extrémité distale de ladite gaine d'homéostase (50) inclut une partie (59) de diamètre constant s'étendant vers ladite vessie de ballonnet (40).

3. Appareil de la revendication 2, caractérisé en ce que la partie à diamètre constant (59) a une longueur d'au moins environ 6,35 mm.

4. Appareil de la revendication 3, caractérisé en ce que ladite gaine d'homéostase (50) a une configuration conique.

5. Appareil de la revendication 1, caractérisé en ce que la gaine d'homéostase (50) a un diamètre externe qui augmente de l'extrémité distale (52) à l'extrémité proximale (54) de ladite gaine d'homéostase (50).

6. Appareil de la revendication 5, caractérisé en ce que ladite gaine d'homéostase (50) a un diamètre interne qui est légèrement supérieur au diamètre externe du cathéter à ballonnet (42) avec un espace (58) entre eux d'au plus environ 50 $\mu$m, au moins à l'extrémité distale (52) de ladite gaine d'homéostase (50).

**Patentansprüche**

1. Intra-Aorta-Ballonvorrichtung mit einem Ballonkatheder (42), der ein proximales und ein distales Ende aufweist sowie eine Ballonblase (40), die aufbtasbar ist und aus der man die Luft ablassen kann, wobei diese Ballonblase abdichtend am distalen Ende des Katheders angebracht ist, umfassend: eine Hämostasis-Hülle (50), die zwischen der Ballonblase (40) und dem proximalen Ende des Ballonkatheters (42) verschiebbar mit dem Ballonkatheder verbunden ist, wobei die besagte Hämostasis-Hülle (50) ein distales Ende (52), an die Ballonblase (40) angrenzend, besitzt sowie Abdichtmittel (60), welche lösbar mit der Hämostasis-Hülle (50) verbunden sind zur Verhinderung von Blutungen zwischen dieser Hämostasis-Hülle (50) und dem besagten Ballonkatheter (42), nachdem die Ballonblase (40) in den Patienten eingeführt worden ist,
dadurch **gekennzeichnet,** daß
die Hämostasis-Hülle (50) einen Außendurchmesser besitzt, der vom distalen Ende (52) zum proximalen Ende (54) der besagten Hämostasis-Hülle (50) allmählich zunimmt.

2. Vorrichtung nach Anspruch 1,
dadurch **gekennzeichnet,** daß
das distale Ende der besagten Hämostasis-Hülle (50) einen Abschnitt (59) konstanten Durchmessers aufweist, der sich in Richtung der Ballonblase (40) erstreckt.

3. Vorrichtung nach Anspruch 2,
dadurch **gekennzeichnet,** daß
der Abschnitt (59) konstanten Durchmessers eine Länge von mindestens 6,35 mm (1/4 inch) aufweist.

4. Vorrichtung nach Anspruch 3,
dadurch **gekennzeichnet,** daß

die Hämostasis-Hülle (50) konisch ist.

5. Vorrichtung nach Anspruch 1,
dadurch **gekennzeichnet,** daß
die Hämostasis-Hülle (50) einen Außendurchmesser aufweist, welcher vom distalen Ende
(52) zum proximalen Ende (54) der
Hämostasis-Hülle (50) größer wird.

6. Vorrichtung nach Anspruch 5,
dadurch **gekennzeichnet,** daß
die Hämostasis-Hülle (50) einen Innendurchmesser besitzt, welcher geringfügig größer ist
als der Außendurchmesser des Ballonkatheters
(42), wobei ein Spielraum (58) dazwischenliegt,
der wenigstens am distalen Ende (52) der
Hämostasis-Hülle (50) nicht grösser ist als
etwa 50 bis 76 μm (2 bis 3-tausendstel inch).

FIG.1

FIG. 3

FIG. 2a

FIG. 2b

FIG. 4

FIG. 5

EP 0 265 864 B1

FIG. 6

FIG. 7

TO WRAP HANDLE

FIG. 8

FIG. 9